Europäisches Patentamt

European Patent Office

Office européen des brevets

(11) Veröffentlichungsnummer: **0 407 332 A1**

# EUROPÄISCHE PATENTANMELDUNG

(21) Anmeldenummer: 90810358.3

(22) Anmeldetag: **15.05.90**

(51) Int. Cl.5: **A61F 2/34**

(30) Priorität: **07.07.89 CH 2541/89**

(43) Veröffentlichungstag der Anmeldung:
**09.01.91 Patentblatt 91/02**

(84) Benannte Vertragsstaaten:
**AT DE FR GB IT**

(71) Anmelder: **GEBRÜDER SULZER AKTIENGESELLSCHAFT**
**Zürcherstrasse 9**
**CH-8401 Winterthur(CH)**

Anmelder: **Protek AG**
**Stadtbachstrasse 64**
**CH-3001 Bern(CH)**

(72) Erfinder: **Giacometti, Roberto, Prof., Dr.-med**
**Centro Ortopedico R Galeazzi, Via R.**
**Galeazzi 4**
**I-20161 Milano(IT)**

(54) Zementfrei im Becken verankerbare Hüftgelenkspfanne.

(57) Die Kontaktfläche (7) einer Hüftgelenkspfanne ist mit einer aus Rillen (12, 13) bestehenden Struktur für das Anwachsen von Knochengewebe versehen. Diese Struktur ist auf den Bereich der Hauptbelastungsrichtungen beschränkt, der sich in der Umgebung von schiefwinklig die Kontaktfläche (7) durchstossenden Verankerungszapfen (5) befindet; die restliche Kontaktfläche (7) ist glatt poliert.

Infolge der Beckenelastizität auftretenden Mikrobewegungen zwischen Knochen und Implantat in wenig oder unbelasteten Bereichen der Kontaktfläche (7) führen auf diese Weise nicht zu Reizungen des am Implantat anliegenden Knochengewebes, da dieses an der glatt polierten Oberfläche praktisch nicht anwächst.

EP 0 407 332 A1

## ZEMENTFREI IM BECKEN VERANKERBARE HÜFTGELENKSPFANNE

Die Erfindung betrifft eine zementfrei im Becken verankerbare Hüftgelenkspfanne, bei der mindestens der Verankerungskörper aus Metall besteht, und aus deren Kontaktfläche zum Knochen, die mit einer das An- und Einwachsen von Knochengewebe fördernden Struktur versehen ist, mindestens ein schiefwinklig zu ihrer Achse stehender Verankerungszapfen hervorsteht.

Die neue Hüftgelenkspfanne kann sowohl als einteilige als auch als zusammengesetzte - d.h. aus einem Verankerungskörper und einem Pfannenkörper mit der eigentlichen Pfannenschale bestehende - Pfanne ausgebildet sein.

Eine Hüftgelenkspfanne, wie sie in ihren konstruktiven Merkmalen vorstehend beschrieben ist, ist bekannt z.B. aus der CH-PS 666 608; diese bekannte Pfanne besteht jedoch nicht aus Metall, sondern aus Kunststoff, der mit einem Metallgitter belegt ist. Bei derartigen Pfannen, bei denen Knochengewebe an die äussere Oberfläche anwächst, hat es sich in der Praxis gezeigt, dass infolge der Elastizität des Beckens Mikrobewegungen zwischen dem Beckenknochen und der ihm zugewandten Kontaktfläche der Pfanne bzw. des Verankerungskörpers auftreten. Diese Bewegungen können vorwiegend in wenig oder unbelasteten Bereichen der Kontaktfläche zu Reizungen und Schädigungen des anwachsenden Gewebes führen, so dass in den genannten Bereichen der Kontaktfläche keine feste Verbindung zwischen Implantat und Knochen entsteht.

Aufgabe der Erfindung ist, derartige Schädigungen des Knochengewebes möglichst zu vermeiden. Diese Aufgabe wird mit der Erfindung dadurch gelöst, dass sich die Oberflächenstruktur auf einen Teilbereich der Kontaktfläche in der Umgebung des Verankerungszapfens beschränkt, und dass ferner die restliche Kontaktfläche glatt poliert ist.

Bei der neuen Ausgestaltung der Kontaktfläche erfolgt ein An- und Einwachsen des Knochens lediglich in den "belasteten" Bereichen der Kontaktfläche, während durch eine polierte Oberfläche auf der restlichen Kontaktfläche eine Verbindung zwischen Knochen und Pfanne möglichst verhindert wird. Es bildet sich dort zwischen beiden lediglich lockeres Bindegewebe, in dem die Pfanne dann "schwinmend gelagert" ist.

Wie experimentelle Untersuchungen gezeigt haben, lässt sich eine günstige Knochentransformation, d.h. eine die Festigkeit der Verbindung zwischen Knochen und Implantat begünstigende Ausbildung der Knochenstruktur, fördern, wenn die Struktur aus konzentrischen, kreisförmigen Rillen besteht, wobei das Zentrum der Kreise in der Mitte zwischen den Verankerungszapfen angeordnet ist, und/oder wenn die Kreisrillen durch entlang ihrer Radien verlaufende, über ihren Umfang verteilte Rillen miteinander verbunden sind.

Bei einer weiteren Ausführungsform, die sich hinsichtlich der Knochentransformation bewährt hat, besteht die Oberflächenstruktur aus Rillen, die sich kreuzen und so ein Rhombusmuster ergeben.

Weiterhin kann der äquatoriale Rand der Pfanne in an sich bekannter Weise im strukturierten Bereich der Kontaktfläche über den Aequator hinaus verlängert sein, wobei der verlängerte Rand bei aus einem Verankerungskörper und einem Pfannenkörper zusammmengesetzten Pfannen sich vorteilhafterweise am Pfannenkörper befindet. Mit diesen an sich bekannten konstruktiven Massnahmen wird vor allem ein Luxieren des Gelenkkopfes aus der eigentlichen Pfannenschale bei "extremen" Bewegungen verhindert.

Im folgenden wird die Erfindung anhand eines Ausführungsbeispiels im Zusammenhang mit der Zeichnung näher erläutert.

Fig. 1 ist ein Meridianschnitt durch eine aus Verankerungskörper und Pfannenkörper zusammengesetzte Hüftgelenkspfanne;

Fig. 2 ist eine Aufsicht auf Fig. 2 von oben;

Fig. 3 gibt eine Aufsicht auf die Pfanne nach Fig. 1 in Richtung der Zapfenachsen wieder;

Fig. 4 zeigt in gleicher Darstellung wie Fig. 3 eine andere Ausführungsform der Oberflächenstruktur auf der Kontaktfläche der Hüftgelenkspfanne.

In einem die Form einer Halbkugelschale aufweisenden Verankerungskörper 1 aus Metall ist ein die Pfannenschale 2 für die Aufnahme eines nicht gezeigten Gelenkkopfes enthaltender Pfannenkörper 3 fixiert. Die Fixierung der beiden Teile 1 und 3 erfolgt in bekannter Weise mit Hilfe eines Schnappverschlusses 4, bei dem ein ringförmiger Ansatz auf der Aussenfläche des Pfannenkörpers in eine entsprechende Nut im Innenhohlraum des Verankerungskörpers 1 einschnappt.

Für die Primärfixation des Verankerungskörpers 1 sind in diesem Verankerungszapfen 5 vorgesehen, die schiefwinklig zur Pfannenachse 6 aus der Kontakfläche 7 des Verankerungskörpers 1 herausragen. Die Zapfen 5, die ebenfalls aus Metall gefertigt sind, können eine rauhe - z.B. durch Sandstrahlen erzeugte - Oberfläche oder eine sonstige Struktur aufweisen, an die Knochengewebe gut anwächst.

Im Polbereich hat der Verankerungskörper 1 eine zylindrische Ausnehmung 9, an die zum Scheitel hin eine zentrale Bohrung 10 anschliesst. Während die Bohrung 10, die ein Gewinde 8 enthält, zur Aufnahme eines nicht gezeigten Setzinstrumentes für den Verankerungskörper dient, bildet die Ausnehmung 9 eine Führung für einen zylindrischen Ansatz 11 im Scheitelbereich des Pfannenkörpers 3. Diese Führung erleichtert dem Operateur ein Einsetzen des Pfannenkörpers 3 in den Verankerungskörper 1.

Die Kontaktfläche 7 trägt eine Oberflächenstruktur, die aus kreisförmigen Rillen 12 und dazu senkrechten Rillen 13 besteht. Erfindungsgemäss ist die Struktur auf einen Bereich in der Umgebung der Verankerungszapfen 5 beschränkt, in dem die Hauptbelastungen bei der Uebertragung von Kräften und Momenten vom Beckenknochen auf den Verankerungskörper 1 auftreten; relativ zu den Zapfen 5 sind die kreisförmigen Rillen 12 dabei so angeordnet, dass ihr Zentrum 16 in die Mitte zwischen den beiden Zapfen 5 zu liegen kommt. Die restliche Oberfläche der Kontaktfläche 7 ist glatt poliert.

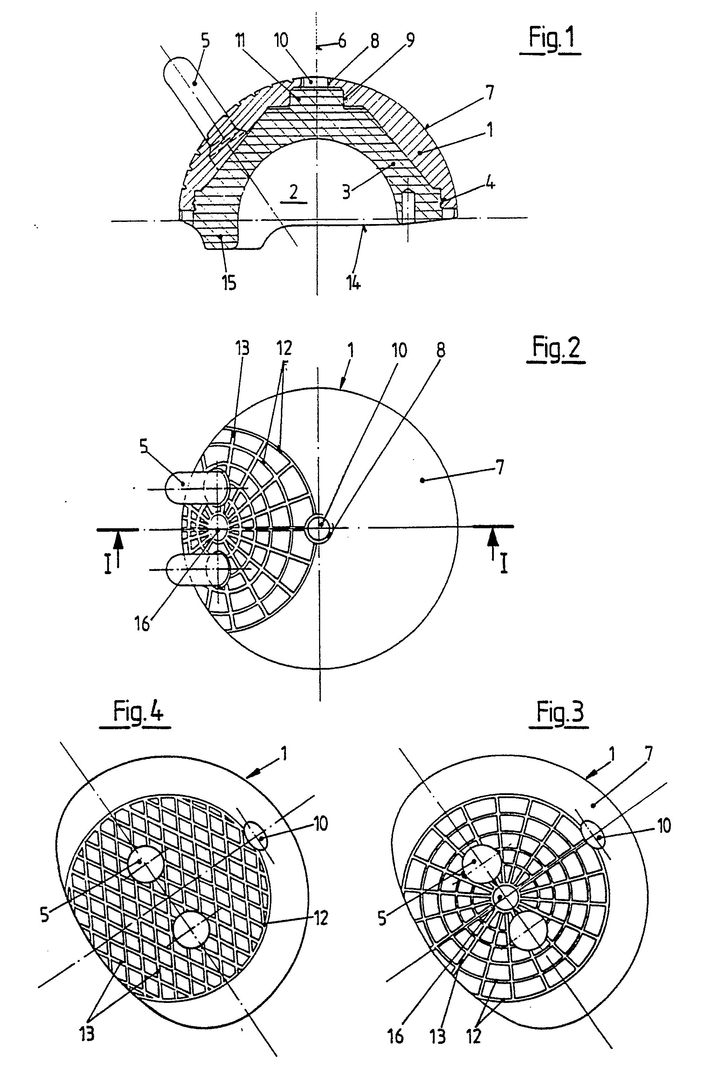

Bei dem Ausführungsbeispiel nach Fig. 4 ist der strukturierte Bereich der Kontaktfläche 7, der durch eine kreisförmige Rille 12 begrenzt ist, von sich kreuzenden geradlinigen Rillen 13 gebildet, so dass ein gitter- oder wabenförmiges Muster entsteht.

Wie Fig. 1 zeigt, ist der Pfannenkörper 3 auf einem Teil seines Umfangs am äquatorialen Rand 14 mit einer an sich bekannten Verlängerung 15 versehen; bei der fertig eingesetzten Pfanne liegt die Verlängerung 15 unterhalb des strukturierten Bereichs und verhindert ein Luxieren des nicht gezeigten Gelenkkopfes bei extremen Bewegungen.

## Ansprüche

1. Zementfrei im Becken verankerbare Hüftgelenkspfanne, bei der mindestens der Verankerungskörper aus Metall besteht, und aus deren Kontaktfläche zum Knochen, die mit einer das An- und Einwachsen von Knochengewebe fördernden Struktur versehen ist, mindestens ein schiefwinklig zu ihrer Achse stehender Verankerungszapfen hervorsteht, dadurch gekennzeichnet, dass sich die Oberflächenstruktur auf einen Teilbereich der Kontaktfläche (7) in der Umgebung des Verankerungszapfens (5) beschränkt, und dass ferner die restliche Kontaktfläche (7) glatt poliert ist.

2. Hüftgelenkspfanne nach Anspruch 1, bei der zwei mit ihren Achsen parallel zueinander verlaufende Verankerungszapfen vorhanden sind, dadurch gekennzeichnet, dass die Struktur aus konzentrischen, kreisförmigen Rillen (12) besteht, wobei das Zentrum (16) der Kreise (12) in der Mitte zwischen den Verankerungszapfen (12) angeordnet ist.

3. Hüftgelenkspfanne nach Anspruch 2, dadurch gekennzeichnet, dass die Kreisrillen (12) durch entlang ihrer Radien verlaufende, über ihren Umfang verteilte Rillen (13) miteinander verbunden sind.

4. Hüftgelenkspfanne nach Anspruch 1, dadurch gekennzeichnet, dass die Oberflächenstruktur aus sich kreuzenden, geradlinigen Rillen (13) besteht.

5. Hüftgelenkspfanne nach einem der Ansprüche 1 bis 4, dadurch gekennzeichnet, dass ihr äquatorialer Rand (14) im Bereich der Struktur über den Äquator hinaus verlängert ist.

6. Hüftgelenkspfanne nach einem der Ansprüche 1 bis 5, dadurch gekennzeichnet, dass sie aus einem metallenen Verankerungskörper (1) und einem Kunststoff-Pfannenkörper (3) besteht.

7. Hüftgelenkspfanne nach Anspruch 5 und 6, dadurch gekennzeichnet, dass der verlängerte Rand (14) in dem Kunststoff-Pfannenkörper (3) angeordnet ist.

Fig.1

Fig.2

Fig.4

Fig.3

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 225 819 (DUTHOIT)(16-06-1987) <br> * Siehe Figuren; Spalte 5, Zeile 62 - Spalte 6, Zeile 5; Anspruch 1 * <br> --- | 1,6 | A 61 F 2/34 |
| A | EP-A-0 149 975 (MATHYS)(31-07-1985) <br> * Siehe das ganze Dokument * <br> --- | 2-4 | |
| A | DE-A-1 806 323 (WALDEMAR LINK)(25-06-1970) <br> --- | | |
| A | EP-A-0 285 756 (SULZER)(12-10-1988) <br> ----- | | |
| | | | **RECHERCHIERTE SACHGEBIETE (Int. Cl.5)** <br><br> A 61 F |

Der vorliegende Recherchenbericht wurde für alle Patentansprüche erstellt

| Recherchenort | Abschlußdatum der Recherche | Prüfer |
|---|---|---|
| DEN HAAG | 10-10-1990 | STEENBAKKER J. |